# EUROPEAN PATENT SPECIFICATION

(11) **EP 3 498 272 B1**
(45) Date of publication and mention of the grant of the patent: **31.01.2024**
(21) Application number: 17860492.2
(22) Date of filing: 10.10.2017
(51) Int. Cl.: A61K 31/216, A61K 9/70, A61K 47/28, A61K 47/32, A61K 47/34, A61P 13/10, A61K 45/06

(54) **OXYBUTYNIN-CONTAINING TRANSDERMAL ABSORPTION PREPARATION**
OXYBUTIN-HALTIGES PRÄPARAT ZUR TRANSDERMALEN ABSORPTION
PRÉPARATION POUR ABSORPTION TRANSDERMIQUE COMPRENANT DE L'OXYBUTYNINE

(30) Priority: 11.10.2016 JP 2016200402
(43) Date of publication of application: 19.06.2019
(73) Proprietor: Hisamitsu Pharmaceutical Co., Inc., Tosu-shi, Saga 841-0017 (JP)
(72) Inventor: KUROKAWA, Takao, Tsukuba-shi Ibaraki 305-0856 (JP); TAKEUCHI, Akio, Tosu-shi Saga 841-0017 (JP)
(74) Representative: Prüfer & Partner mbB Patentanwälte · Rechtsanwälte
(86) International application number: PCT/JP2017/036619
(87) International publication number: WO 2018/070370

(56) References cited:
- WO-A1-2011/136283
- WO-A1-2013/061969
- WO-A1-2013/061969
- JP-A- 2013 248 182
- JP-A- 2014 125 466

## Description

### Technical Field

The present invention relates to a transdermal absorption preparation (patch) that contains oxybutynin.

Priority is claimed on Japanese Patent Application No. 2016-200402, filed on October 11, 2016.

### Background Art

A patch by which a medication is administered from the skin has merits in that: it is possible to avoid a rapid rise in a blood concentration of the medication; it is easy to maintain medication absorption; it is possible to avoid a first pass to the liver; it is possible to discontinue dosing when a side effect develops; and the like, compared to injections and oral preparations. On the other hand, in a case where the medication was administered by application of the patch, skin irritations such as pruritus, rash, pain, eczema, and dermatitis occurred at the application site in some cases.

PTL 1 describes a transdermal absorption preparation (patch) which contains cholesterols which is recognized for a sufficient effect of reducing skin irritation toward a wide range of medications.

Among such patches, since a patch using a fabric or the like for a support is excellent in flexibility and elasticity and easy to follow the shape change of the skin, the patch is excellent in adhesion. For the patch having such characteristics and using a support such as a fabric, cholesterols having the effect of reducing skin irritation are desired to be mixed therein.

PTL 2 describes that in a transdermal absorption preparation (patch) in which a PET fabric is used for a support and cholesterols are mixed therein, adhesion decreases due to the contained cholesterols. It is also described that when containing polyisobutylene (PIB) as an adhesive base, it is possible to improve the adhesion. Since it may be difficult to use PIB as the adhesive base depending on a medication to be used, another method to improve the adhesion is desired.

### Citation List

### Patent Literature

[PTL 1] PCT International Publication No. WO2011/136283
[PTL 2] PCT International Publication No. WO2013/061969

### Summary of Invention

### Technical Problem

In addition to the above, the present inventors have found that if cholesterols are mixed in the patch using base fabric for a support, fluidity of an adhesive composition increases, and when stored under severe conditions, releasability of a medication may change over time. In the patch, since the releasability greatly influences a therapeutic effect of the medication, it is important for the patch to maintain a predetermined releasability of the medication even after storage.

An object of the present invention is to maintain adhesion despite an increase in the fluidity of the adhesive composition and to reduce a change in releasability of medication over time after storage, in a patch using a fabric or the like for a support and containing cholesterols which are recognized for an effect of reducing skin irritation.

### Solution to Problem

The object is achieved by the oxybutynin-containing transdermal absorption preparation according to claim 1. Refinements of the invention are specified in the dependent claims.

The present inventors conducted intensive studies to achieve the object, found that in a case where a part of a support layer formed of a base fabric has water repellency, even in a case of being stored under severe conditions, it is possible to reduce a change in the releasability of the medication over time, and completed the invention of the present application.

An oxybutynin-containing transdermal absorption preparation (a patch) according to a first aspect of the present invention includes: a support layer that is formed of a base fabric and has a water-repellent surface formed by a water-repellent treatment with a fluorine-containing compound in at least one side thereof in a thickness direction; and an adhesive layer laminated on one side of the support layer, in which the adhesive layer includes an adhesive composition containing oxybutynin or a pharmaceutically acceptable salt thereof, cholesterols, and a rubber adhesive base.

According to a second aspect of the present invention, in the oxybutynin-containing transdermal absorption preparation (patch) of the first aspect, the oxybutynin may be oxybutynin hydrochloride.

According to a third aspect of the present invention, in the oxybutynin-containing transdermal absorption preparation (patch) of any one of the first to second aspects, the base fabric may be a knitted fabric.

According to a fourth aspect of the present invention, in the oxybutynin-containing transdermal absorption preparation (patch) of any one of the first to third aspects, the rubber adhesive base may contain a styrene-isoprene-styrene block copolymer.

According to a fifth aspect of the present invention, in the oxybutynin-containing transdermal absorption preparation (patch) of any one of the second to fourth aspects, a content percentage of the oxybutynin may be 4% to 50% by mass relative to the adhesive composition, and the cholesterols may be cholesterol and a content percentage thereof may be 0.05 to 10% by mass relative to the adhesive composition.

### Advantageous Effects of Invention

According to the invention, it is possible to reduce a change in adhesion and releasability of medication over time, in an oxybutynin-containing transdermal absorption preparation (patch) using a fabric or the like for a support and containing cholesterols which are recognized for an effect of reducing skin irritation.

### Brief Description of Drawings

FIG. 1 is a sectional diagram of an oxybutynin-containing transdermal absorption preparation (patch) in a thickness direction, according to an embodiment of the present invention.
FIG. 2 is a graph showing a relationship between a release rate and a release time of a medication.
FIG. 3 is a graph showing a relationship between a release rate and a release time of a medication.
FIG. 4 is a graph showing a relationship between a release rate and a release time of a medication.
FIG. 5 is a graph showing a relationship between a permeation rate and a release time of a medication.
FIG. 6 is a graph showing a relationship between a thickness maintenance rate and a maximum permeation rate maintenance rate.

### Description of Embodiments

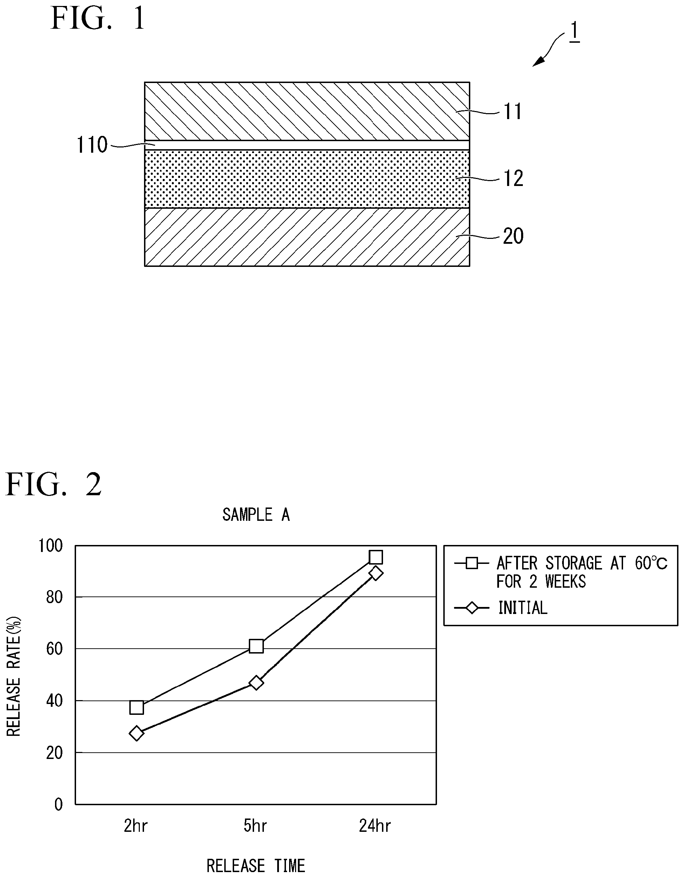

Hereinafter, a patch (oxybutynin-containing transdermal absorption preparation) according to an embodiment of the present invention will be described with reference to FIG. 1. The patch (oxybutynin-containing transdermal absorption preparation) 1 of the present embodiment is configured to include a support layer 11 formed of a base fabric. Since the patch is excellent in flexibility and elasticity and easy to follow the shape change of the skin, it can be used in various fields and applications such as medical use and home use.

FIG. 1 is a sectional diagram of the patch 1 in a thickness direction, according to the present embodiment. The patch 1 includes the support layer 11 and an adhesive layer 12 laminated on one side of the support layer 11.

### (Support Layer)

The support used for the support layer 11 may be anything suitable for supporting the adhesive layer 12, and is preferably a base fabric excellent in elasticity. Polyolefins such as polyethylene, polypropylene, and polybutadiene, polyesters such as ethylene vinyl acetate copolymer, polyvinyl chloride, polyethylene terephthalate, polyethylene naphthalate, and polybutylene terephthalate, polyamides such as nylon, silk, animal hair (for example, wool), and cellulose such as polyurethane and cotton or laminates or mixtures thereof may be used, and these can be used as a porous material, a foamed material, a woven fabric, a nonwoven fabric, a knitted fabric, a laminated product thereof, and the like. A weave of the woven fabric or a knitting of the knitted fabric is not particularly limited, and examples thereof include warp knitting (tricot knitting, denby tricot knitting, satin knitting, atlas knitting, flat knitting, rib knitting, and pearl knitting) and circular knitting (double-sided stockinette knitting, stockinette knitting, and fleece knitting). The nonwoven fabric is not particularly limited, and may be a spunlace nonwoven fabric, a thermal bond nonwoven fabric, a spunbonded nonwoven fabric, and the like.

Here, a fabric weight of the base fabric may be 20 to 200 g/m², and may be 70 to 140 g/m². In addition, a thickness of the base fabric may be 0.1 to 3 mm.

A surface of the adhesive layer 12 of the patch 1 on a side opposite to a side in contact with the support layer 11 may include a releasable coating 20 to be removed by peeling off before application to an affected area. As the releasable coating 20, a film or a sheet formed of polyolefins such as polyethylene and polypropylene, polyesters such as polyethylene terephthalate, and celluloses such as paper and the film or the sheet which is release-treated with silicone can be used.

The support layer 11 has a water-repellent surface 110 formed by a water-repellent treatment in at least one side thereof in a thickness direction. The water-repellent surface 110 is subjected to the water-repellent treatment with a fluorine-containing compound having a high water- and oil-repellent effect. The water-repellent surface 110 may be provided on a surface where the support layer 11 is in contact with the adhesive layer 12, may be provided on a surface on a side opposite to the surface where the support layer 11 is in contact with the adhesive layer 12, or may be provided throughout the support. The water-repellent surface 110 may be formed over the entire surface of the support layer 11 in a planar direction or may be formed only in a region which is in contact with the adhesive layer 12.

In addition, a water-repellent surface may be formed on an intermediate layer of the support layer 11. For example, in a case where the support layer 11 is formed by overlapping two base fabrics, the water-repellent surface can be formed therebetween.

Here, a portion which is formed on the support layer 11 and has water repellency may be not a planar shape unlike the water-repellent surface 110, and may be a three-dimensional shape formed in a part of the support layer 11. A spot-like water-repellent portion formed in both the thickness direction and the surface direction in the support layer 11, or a streaky water-repellent portion may be adopted. The water-repellent portion may be formed on a part of a knitting pattern in accordance with the knitting pattern of the base fabric.

In addition, the base fabric may be formed from a material which has been subjected to a water-repellent treatment in advance. When the support layer 11 is formed using a base fabric in which all or a part thereof is formed using water-repellent-treated filament or thread, the support layer 11 can have water repellency.

That is, the surface of the support layer 11 has water repellency.

For the water-repellent treatment, for example, the base fabric may be formed from a material which has been subjected to the water-repellent treatment in advance, or the water-repellent treatment may be carried out after the base fabric is formed. The water-repellent treatment can impart water repellency by reacting or attaching a water-repellent agent, which is a fluorine-containing compound to a fiber or the base fabric. More specifically, the water repellency can be imparted by spraying a solution or an emulsion containing the water-repellent agent to the fiber or the base fabric, or by immersing the fiber or the base fabric in a solution containing the water-repellent agent. When performing the immersing, it is possible to make the entire fiber or the base fabric water-repellent, which is more preferable. The water-repellent agent is not particularly limited, and specific examples thereof include, as fluorine-based agents, Asahi Guard E (trade name, Asahi Glass), NK Guard S (trade name, Nicca Chemical), Unicapolone (trade name, Union Chemistry Industry), TS guard (trade name, Tokai Refinery), Dickguard (trade name, Dainippon Ink), and the like.

In order to confirm whether or not at least a part of the support layer 11 has water repellency, for example, as a simplified method, when a patch is held horizontally such that a base fabric side faces upward and water droplets of purified water are gently dropped onto the base fabric, it may be determined whether or not water is retained in a water drop shape as it is, on the base fabric. It can also be determined by using an instrument such as a micropipette in which when dropping 20 droplets of purified water at 20°C determined the mass thereof is set to 0.90 to 1.10 g in accordance with general rules of the Japanese Pharmacopoeia.

When at least a part of the support layer 11 has water repellency, it brings about a favorable effect on a change in adhesion of the adhesive composition laminated as the adhesive layer 12 or releasability of a medication over time. Details will be described later.

When forming the water-repellent surface 110 over the entire area of the surface where the support layer 11 is in contact with the adhesive layer 12, it is possible to bring out a more favorable effect on the change in the adhesion of the adhesive composition or the releasability of the medication over time.

### (Adhesive Layer)

The adhesive layer 12 includes an adhesive composition containing a medication exhibiting a medicinal effect, cholesterols, and lipophilic adhesive base. The cholesterols which are recognized for a sufficient effect of reducing the skin irritation toward a wide range of medications are mixed in the adhesive composition as a skin irritation suppressant.

### (Medication)

From the viewpoint of reducing the skin irritation, examples of the medication mixed in the adhesive composition include one or more basic medications selected from the group consisting of oxybutynin, tolterodine, asenapine, bisoprolol, risperidone, nicotine, and citalopram, or a pharmaceutically acceptable salt thereof; and a pharmaceutically acceptable salt thereof. According to the invention, oxybutynin is present in the adhesive layer. Among these, in a case where the oxybutynin hydrochloride is selected as the medication, the effect of the present embodiment is particularly exhibited.

In the adhesive composition, in addition to the above medication, an antihypertensive agent such as atenolol, amlodipine, and captopril, a vasodilator such as isosorbide nitrate and nitroglycerin, a warming sensation agent such as capsaicin, capsicum extract, capsicum powder, capsicum tincture, and nonylic acid vanillylamide, a nonsteroidal antiinflammatory drug such as indomethacin, camphor, ketoprofen, methyl salicylate, glycol salicylate, diclofenac sodium, flurbiprofen, felbinac, meloxicam, and loxoprofen, a hormonal agent such as estradiol, norethisterone, and estriol, an antihistamine such as ketotifen, anti-alzheimer's drugs such as memantine and donepezil, an antidepressant such as sertraline, fluoxetine, paroxetine, citalopram, and fluvoxamine, a therapeutic medication for gastric ulcer such as teprenone, a therapeutic medication for overactive bladder such as solifenacin, a bronchodilator such as tulobuterol, a cooling sensation agent such as menthol and peppermint oil, a therapeutic medication for parkinson's disease such as pergolide and rotigotine, a vitamin preparation such as retinoid, and the like can be mixed therein.

When mixing the skin irritation suppressant, skin irritation due to these medications or medicinal ingredients is also reduced. For the medication contained in the adhesive layer 12 of the patch 1 of the present embodiment, any of these medications can be selected. In addition, medications other than the medications exemplified here may be selected. According to the invention, oxybutynin is present.

Among the above medications to be selected, oxybutynin hydrochloride is a medication used for a treatment of "overactive bladder" having symptoms such as urinary urgency and frequent urination. In the related art, oxybutynin hydrochloride is mainly administered orally, and side effects such as mouth dryness, constipation, and drowsiness caused by metabolites due to a first-pass liver effect are known. A patch containing the oxybutynin hydrochloride is used to reduce the side effects caused by oral administration. Oxybutynin hydrochloride may develop skin irritation such as pruritus, erythema, rash, pain, eczema, and dermatitis rarely when administered from the skin, in some cases. Therefore, it is one of the medications that are desired to be mixed with the skin irritation suppressant of cholesterols.

A chemical name of oxybutynin (oxybutynin) is α-phenylcyclohexane glycolic acid 4-(diethylamino)-2-butynyl. The oxybutynin used in the present embodiment is not limited to the hydrochloride salt and may be free form oxybutynin, may be another pharmaceutically acceptable oxybutynin salt, or may be a combination thereof. The pharmaceutically acceptable salt of the oxybutynin may be an inorganic acid salt or an organic acid salt, and in addition to the above, for example, the hydrochloride salt, an inorganic acid salt such as a hydrobromide salt or a silicate and organic acid salts such as an acetate salt, citrate, fumarate, and maleate are known.

A content of the medication in the patch varies depending on the kind of the medication, an illness to be treated, a dosing method, a composition of the adhesive composition, and the like, but is preferably 4% to 50% by mass, may be 5% to 30% by mass, and may also be 8% to 17% by mass, based on the total amount of the adhesive composition. In a case where the medication is less than 4% by mass, sufficient therapeutic effect tends to be difficult to obtain. In a case where the content of medication is in the above ranges, skin irritation due to the patch tends to be relaxed or less, it is easy to obtain the targeted therapeutic effect, and the adhesion of the patch is also likely to be appropriate. In a case where the content of the medication exceeds 50 mass%, skin irritation due to the patch tends to be strong. In addition, in a case where the medication is oxybutynin, when converted as the hydrochloride as needed, the content thereof may be 5% to 30% by mass, desirably 8% to 17% by mass, based on the total amount of the adhesive composition.

Various studies on an immune reaction of epidermal cells have been conducted as one of mechanisms to express the skin irritation of the medication. The epidermal cells play a central role in skin immunity by: releasing a number of inflammation-inducing substances such as cytokines, chemokines, inflammatory mediators, and cell growth factors to an outside of the cell; or expressing the cytokine receptor, adhesion factor, and MHC class II on the cell (Skin immunity handbook (Chinese and foreign medical society)).

Examples of the inflammation-inducing substances released by the epidermal cells include interleukin (IL)-1α, IL-10, IL-12, IL-18, TNF-α, GM-CSF, IL-6, IL-7, IL-15, TGF-α, amphiregulin, HB-EGF, bFGF, VEGF, PDGF, SCF, IFN-β, IFN-γ, TGF-β, MIP-3α, IP-9, IP-10, Mig, IL-8, GROα, RANTES, MCP-1, TARC, prostaglandin, leukotriene, substance P, reactive oxygen species, and nitrogen oxides. These are very diverse and interact with each other complexly to regulate the immune response.

Therefore, in the present specification, the skin irritation reduction refers to a decrease of production of so-called skin irritation mediators, such as prostaglandin E2 (PGE 2), IL-1α, IL-6, IL-8, due to the medication in an in vitro test using the epidermal cells, and/or reduction of skin irritation such as erythema or scab of skin and edema formation due to the medication, in a case of in vivo. The skin irritation can be evaluated, for example, by Primary Irritation Index (PII).

### (Skin Irritation Suppressant)

As the skin irritation suppressant to be mixed in the adhesive composition, cholesterols selected from cholesterol, a cholesterol derivative, and a cholesterol analogue are used. A mixing amount thereof is preferably 0.05% or more by mass based on the total amount of the adhesive composition.

The cholesterols are alcohols having a steroid skeleton selected from cholesterol, a cholesterol derivative, and a cholesterol analogue. Cholesterol in a narrow sense is (3β)-cholesta-5-ene-3-ol cholesta-5-ene-3β-ol, which is known as an essential component of a cell membrane of higher animals. The cholesterol derivative refers to natural or synthetic cholesterol derived from animals and plants, microorganisms, and fungi, and examples thereof include acyl cholesterol, which is an ester form in which a fatty acid is bonded to a hydroxy group moiety. In addition, the cholesterol analogue refers to natural or synthetic cholesterol analogue, and examples thereof include phytosterol derived from plant cells, such as sitosterol, stigmasterol, fucosterol, spinasterol, campesterol, and brassicasterol, and ergosterol derived from fungi. One kind of these can be used alone, and two or more thereof can be used by being mixed.

Among the cholesterols, the cholesterol is preferable, and cholesterol derived from wool is more preferable. The cholesterol, the cholesterol derivative, and the cholesterol analogue are all classified as steroids, and among them, belong to a subgroup called sterol (steroid alcohol). The adhesive composition may contain any one or a combination of two or more of these cholesterols. These cholesterols have an action of reducing the skin irritation due to the medication such as oxybutynin. Therefore, it is possible to reduce the skin irritation due to the medication such as oxybutynin by mixing these compounds therein.

A mixing amount of the cholesterols is preferably 0.05% or more by mass based on the total amount of the adhesive composition and, for example, may be 0.05% by mass to 30% by mass, 0.1% to 25% by mass, 0.5% to 20% by mass, and 1% to 15% by mass. However, depending on the kind of the adhesive composition, when the cholesterols are mixed therein in a mixing amount of more than 15% by mass, the adhesion of the patch may not be sufficiently obtained. Therefore, the mixing amount may be 15% by mass or less and is more preferably 10% by mass or less.

The cholesterols to be mixed as the skin irritation suppressant are substances each having a melting point higher than 100°C, and are naturally solid at a normal temperature (1 to 30°C). Thus, even when these are mixed in the adhesive composition, it was predicted that no change in which the fluidity (plasticity) of the adhesive composition is enhanced will occur. However, unexpectedly, when the cholesterols are mixed in the adhesive composition, it was found that the fluidity (plasticity) of the adhesive composition increased.

In a case where the base fabric is used as the support layer and the cholesterols are mixed as the skin irritation suppressant, the fluidity of the adhesive composition remarkably increases. When storing under severe conditions or when storing for a long period of time even at a normal temperature and normal pressure, phenomenons in which the adhesive composition gradually penetrates into the support layer, the adhesion of the patch decreases greatly, and the releasability of the medication changes may occur in some cases. These phenomenons become remarkable as the mixing amount of the cholesterols increases.

For example, a patch in which an adhesive composition containing cholesterols mixed therein is laminated on a base fabric formed by stockinette-knitting polyester has approximately 50% of adhesion after being prepared and stored at 60°C for two weeks, compared to a case where the cholesterols are not mixed therein. In addition, the releasability of the medication in a case where the release time is 5 hours may increase approximately twice, compared to a case where the cholesterols are not mixed therein.

In the patch 1, since the releasability greatly influences the therapeutic effect of the medication, it is important for the patch 1 to maintain the predetermined releasability even after storage. In the patch 1 using the fabric for the support layer 11 and having cholesterols, in which an effect of reducing the skin irritation is recognized, mixed therein, a water-repellent portion is provided on at least a part of the support layer 11, in order to reduce changes in the adhesion and the releasability of the medication after storage. For example, in the support layer 11 formed of the base fabric, the water-repellent surface 110 is provided on at least one side thereof in a thickness direction. The water-repellent surface 110 may be subj ected to the water-repellent treatment with a silicone compound having a high water-repellent effect, or may be subjected to the water-repellent treatment with a fluorine compound having a high water- and oil-repellent effect. The water-repellent treatment with the fluorine compound also having an oil-repellent effect is more suitable. When using the support layer 11 having the water-repellent portion such as the water-repellent surface 110 on a part thereof, it is possible to suppress the fluidity of the cholesterols mixed in the adhesive layer 12, and in the patch 1 when stored under severe conditions, it is possible to maintain the adhesion and releasability of the medication.

### (Transdermal Absorption Enhancer)

The adhesive composition may further contain a transdermal absorption enhancer which is a component for regulating transdermal absorbability of the medication. The transdermal absorption enhancer which can be used may be any of compounds having a transdermal absorption-promoting action for the skin used in the related art. Examples thereof include organic acids, fatty acids having 6 to 20 carbon atoms, fatty alcohol, fatty acid ester, amide, ethers, aromatic organic acid, aromatic alcohol, and aromatic organic acid ester and ether (which may be either saturated or unsaturated and may be any of cyclic, linear, or branched), lactic acid esters, acetic acid esters, a monoterpene compound, a sesquiterpene compound, Azone, Azone derivatives, pyrrothiodecane, glycerin fatty acid esters, propylene glycol fatty acid esters, sorbitan fatty acid esters (Span type), polysorbate type (Tween type), polyethylene glycol fatty acid esters, polyoxyethylene hydrogenated castor oil type (HCO type), polyoxyethylene alkyl ethers, sucrose fatty acid esters, and vegetable oil.

Examples of such organic acids include aliphatic (mono, di, or tri) carboxylic acid (such as acetic acid, propionic acid, citric acid (including anhydrous citric acid), isobutyric acid, caproic acid, caprylic acid, lactic acid, maleic acid, pyruvic acid, oxalic acid, succinic acid, and tartaric acid), aromatic carboxylic acid (such as phthalic acid, salicylic acid, benzoic acid, and acetylsalicylic acid), alkyl sulfonic acid (such as methanesulfonic acid, ethanesulfonic acid, propyl sulfonic acid, butanesulfonic acid, polyoxyethylene alkyl ether sulfonic acid), and an alkyl sulfonic acid derivative (N-2-hydroxyethylpiperidine-N'-2-ethanesulfonic acid), a cholic acid derivative (such as dehydrocholic acid), or a salt thereof (for example, an alkali metal salt such as a sodium salt). Among these organic acids, the carboxylic acids and salts thereof are preferable, and acetic acid, sodium acetate, and citric acid are particularly preferable. One kind of these organic acids may be used alone and two or more kinds thereof may be used in combination.

In addition, examples of other transdermal absorption enhancers can include lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methyl laurate, hexyl laurate, lauric acid diethanolamide, isopropyl myristate, myristyl myristate, octyldodecyl myristate, cetyl palmitate, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneo roll, d-limonene, isoeugenol, isoborneol, nellol, dl-camphor, glycerin monocaprylate, glycerin monocaprate, glycerin monolaurate, glycerin monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, polyoxyethylene hydrogenated castor oil, pyrrothiodecane, and olive oil. Among these, the oleyl alcohol, the lauryl alcohol, the isostearyl alcohol, the lauric acid diethanolamide, the glycerin monocaprylate, the glycerin monocaprate, the glycerin monooleate, the sorbitan monolaurate, the propylene glycol monolaurate, the polyoxyethylene lauryl ether, and the pyrrothiodecane are particularly preferable. Among these, the fatty acids having 6 to 20 carbon atoms are preferable, and the oleic acid is particularly preferable. Two or more kinds of these transdermal absorption enhancers may be used by being mixed. In a case where the adhesive composition contains the transdermal absorption enhancer, the fluidity of the adhesive composition is likely to increase.

In the adhesive composition, a mass ratio between the medication and the cholesterols may be 400:1 to 1: 10, 300:1 to 1:5, and 150:1 to 1:1, and may further be 15:1 to 1:1. According to the mass ratio therebetween, it is possible to reduce the skin irritation without affecting the skin permeability of the medication such as oxybutynin.

The adhesive layer 12 of the patch preferably substantially does not contain water. Substantially containing no water means that the adhesive layer 12 is formed of non-aqueous material. However, it is permitted that the adhesive layer 12 contains a trace amount of moisture of 5% by mass or less derived from a raw material or the manufacturing environment.

### (Adhesive Base)

As the adhesive base, a rubber adhesive base is used from the viewpoint of adhesion and less irritation to the skin.

Examples the rubber adhesive base include a styrene-isoprene copolymer, a styrene-isoprene-styrene block copolymer (SIS), polyisoprene, polyisobutylene (PIB), butyl rubber, a styrene-butadiene copolymer, a styrene-butadiene-styrene block copolymer (SBS), styrene-butadiene rubber (SBR), natural rubber, polybutadiene, and polysiloxanes. Among these, SIS, PIB, and polysiloxanes are preferable, and from the viewpoints of adhesiveness, stretchability, and cohesion, the SIS and the PIB are preferable.

In particular, the SIS has pseudo-crosslinking characteristics by a styrene domain, and can be used as the adhesive base to which hot melt coating can be performed. Therefore, the adhesive base using the SIS is excellent in adhesive force and holding power, and is also suitable for combination with the support layer formed of the base fabric excellent in elasticity. The SIS may be 5% to 60% or may be 10% to 35%, in terms of mass ratio of styrene in a copolymer. A molecular weight of the SIS may be 20000 to 500000 or may be 50000 to 350000. In addition, the mass ratio of the diblock structure in the SIS may be 50% or less, or may be 35% or less.

Two or more kinds of the above adhesive bases may be used by being mixed. In consideration of formation of the adhesive layer 12 and sufficient skin permeability of the medication such as oxybutynin, a mixing amount of the adhesive base is generally 5% to 90% by mass based on the total amount of the adhesive composition, and may be 10% to 70% by mass, 10% to 50% by mass, and further 10% to 30% by mass.

### (Plasticizer)

The adhesive layer 12 may further contain a plasticizer. Examples of the plasticizer can include petroleum oil such as paraffinic process oil, naphthenic process oil, and aromatic process oil; squalane; squalene; vegetable oil such as olive oil, camellia oil, castor oil, tall oil, and peanut oil; silicone oil; dibasic acid ester such as dibutyl phthalate and dioctyl phthalate; liquid rubber such as polybutene and liquid isoprene rubber; liquid fatty acid ester such as isopropyl myristate, hexyl laurate, diethyl sebacate, and diisopropyl sebacate; diethylene glycol; polyethylene glycol; glycol salicylate; propylene glycol; dipropylene glycol; triacetin; triethyl citrate; and crotamiton. Among these, liquid paraffin, liquid polybutene, the isopropyl myristate, the diethyl sebacate, and the hexyl laurate are preferable, and the liquid polybutene, the isopropyl myristate, and liquid paraffin are particularly preferable. Two or more kinds of these plasticizers may be used by being mixed.

In consideration of sufficient skin permeability of oxybutynin and maintenance of sufficient cohesion as the patch 1, a mixing amount of the plasticizer is generally 10% to 70% by mass based on the total amount of the adhesive composition, and may be 10% to 60% by mass and further 10% to 50% by mass. As the adhesive composition contains a lot of the plasticizers, the fluidity of the adhesive composition is likely to increase.

### (Tackifying Resin)

The adhesive layer 12 may further contain a tackifying resin, which is a component to regulate the adhesiveness of the adhesive composition to the skin. Examples of the tackifying resin include rosin, a rosin derivative such as glycerin ester of rosin, hydrogenated rosin, glycerin ester of hydrogenated rosin, and pentaerythritol ester of rosin, alicyclic saturated hydrocarbon resins such as Alcon P 100 (trade name, Arakawa Chemical Industries, Ltd.), aliphatic hydrocarbon resin such as Quinton B 170 (trade name, Zeon Corporation), terpene resin such as Clearon P-125 (trade name, Yasuhara Chemical Co., Ltd.), maleic acid resin. Among these, the glycerin ester of hydrogenated rosin, the alicyclic saturated hydrocarbon resin, the aliphatic hydrocarbon resin, and the terpene resin are particularly preferable.

In consideration of sufficient adhesion as the patch 1 and irritation to the skin at the time of peeling, a mixing amount of the tackifying resin is generally 5% to 70% by mass based on the total amount of the adhesive composition, and may be 5% to 60% by mass and further 10% to 50% by mass.

### (Manufacturing Method)

Next, a manufacturing method of the patch 1 will be described. Here, a manufacturing method of the patch 1 including a support layer 11 that is formed of a base fabric and includes the water-repellent surface 110 in at least one side thereof in a thickness direction will be described. First, the water-repellent treatment is carried out on at least one side of the base fabric in the thickness direction to form the water-repellent surface 110, and the support layer 11 is formed. The water-repellent surface 110 is subjected to the water-repellent treatment with a fluorine compound having a high water- and oil-repellent effect. The whole base fabric may be subjected to the water-repellent treatment by immersing in a solution containing a water-repellent treating agent, and then taking out and drying (a support layer preparation step)

In a case of the patch 1 using the rubber adhesive base, using a mixing machine such as a kneader and a mixer, the adhesive base, the medication, and the cholesterols, and optionally the plasticizer, the tackifying resin, and other additives are added, heated, and melted to be spread directly on the support layer 11 and then laminated by pressure bonding to the support layer 11. Alternatively, the melt is spread onto a paper or film once subjected to a release treatment and then laminated by pressure bonding to the support layer 11. Subsequently, the laminate is cut to have an appropriate size to obtain the patch 1.

The patch 1 having a configuration as described above can also be manufactured by other methods which are commonly known. For example, an adhesive composition containing oxybutynin is thermally melted, applied to a release paper or the support layer 11, and then pasted to the support layer 11 or the release paper to obtain this preparation. Alternatively, an adhesive composition component containing oxybutynin is dissolved in a solvent such as toluene, hexane, or ethyl acetate, and spread on a release paper or the support layer 11. The solvent is dried and removed, and then it is possible to obtain this preparation pasted with the support layer or the release paper.

To these patches 1, various pharmacologically acceptable additives such as a stabilizer, an antioxidant, a fragrance, a filler, and a transdermal absorption enhancer can be added within a range not to impair the object of the present invention.

An experiment conducted to evaluate an effect of adhesion (adhesiveness) of a patch depending on differences such as cholesterols inclusion, and results thereof will be described.

### (Experiment 1) Adhesion Evaluation

### (1-1 Preparation of Sample)

The following four kinds of samples were prepared (Samples A, B, C, and D). Sample A is a patch in which an adhesive composition containing no cholesterols was laminated on the support layer formed of the base fabric. Sample B is a patch in which an adhesive composition containing 3% by mass of cholesterols relative to the total amount of 100% by mass of the adhesive composition was laminated on the support layer formed of the base fabric. Sample C is a patch 1 in which the adhesive composition containing 3% by mass of cholesterols relative to the total amount of 100% by mass of the adhesive composition was laminated on the water-repellent surface 110 containing a fluorine compound, the water-repellent surface 110 being provided on the support layer 11 formed of the base fabric. Sample D is a patch 1 in which the adhesive composition containing 3% by mass of cholesterols relative to the total amount of 100% by mass of the adhesive composition was laminated on the water-repellent surface 110 containing a fluorine compound, the water-repellent surface 110 being provided on the support layer 11 formed of the base fabric, as Sample C. In Sample D, a content percentage of oxybutynin hydrochloride as the medication is set to 10% by mass. The water-repellent surface 110 of each of Samples C and D is provided on the entire surface where the base fabric is in contact with the adhesive composition of the adhesive layer 12.

All adhesive compositions laminated as the adhesive layer contain the oxybutynin hydrochloride as the medication and also contain lipophilic adhesive base. Table 1 shows a composition table of the adhesive composition laminated on each sample. In the table, "Other additives" are additives including a component regulating absorbability of the medication, a component regulating adhesiveness, or the like.

Respective components were mixed to prepare a solution, and a solvent was optionally added thereto. The solution was cast on a polyethylene terephthalate (PET) film which had been subjected to a release treatment, and then, the solvent was dried to form an adhesive layer (140 g/m²). Further, in Samples A and B, the base fabric having no water-repellent surface was set as the support layer and the adhesive layer was laminated. In Samples C and D, the base fabric having the water-repellent surface 110 was set as the support layer 11, and the adhesive layer 12 was laminated. The base fabric was made of a polyester material such as PET, and a knitted fabric obtained by stockinette knitting with a fabric weight of 100 g/m² was used. The fabric was cut appropriately, and was hermetically wrapped in an aluminum laminate packaging material.

**[Table 1]**

| COMPONENTS [% BY MASS] | SAMPLE A | SAMPLE B | SAMPLE C | SAMPLE D |
|---|---|---|---|---|
| OXYBUTYNIN HYDROCHLORIDE | 15.0 | 15.0 | 15.0 | 10.0 |
| SIS BLOCK COPOLYMER | 17.2 | 10.2 | 10.2 | 11.4 |
| TACKIFYING RESIN | 40. 1 | 38.1 | 38.1 | 43.0 |
| LIQUID PARAFFIN | 14.3 | 13.6 | 13.6 | 15.3 |
| CHOLESTEROL | 0.0 | 3.0 | 3.0 | 3.0 |
| OTHER ADDITIVES | 13.4 | 20.1 | 20.1 | 17.3 |
| TOTAL | 100 | 100 | 100 | 100 |

### (1-2 Experimental Procedure)

The adhesion was measured by a probe tack method (JIS Z 0237). Measurement was performed after preparation of a sample patch (hereinafter, referred to as "initial state") and after storage at 60°C for 2 weeks by being hermetically wrapped in an aluminum laminate packaging material (hereinafter, referred to as "after storage"). For the two measurements with different conditions, separate samples were used. A case where the adhesion after storage decreased by 10% or more with respect to the adhesion of the initial state was determined as "decreased over time".

### (1-3 Results)

The results are shown in Table 2. In the initial state, the adhesion in Samples B and C decreased compared to Sample A. It was observed that the adhesion decreased in the initial state by containing cholesterols. After storage, the adhesion of Sample B remarkably decreased. In Sample C provided with the water-repellent surface 110, the adhesion after storage improved more than Sample B. It is considered that when using the support layer 11 having the water-repellent surface 110, it is possible to suppress the fluidity of the adhesive composition due to the cholesterols mixed in the adhesive layer 12 and to maintain the adhesion of the patch 1 after storage.

In addition, even in Sample D in which a content percentage of the medication was set to 10%, it is possible to maintain the adhesion even after storage, without change from the initial state. It is considered that, even in a case where the content percentage of the medication was adjusted in accordance with a therapeutic effect, the effect of suppressing the fluidity of the adhesive composition due to the cholesterols was achieved by providing the water-repellent surface 110.

**[Table 2]**

| | | SAMPLE A | SAMPLE B | SAMPLE C | SAMPLE D |
|---|---|---|---|---|---|
| **ADHESION** [gF] | INITIAL STATE | 184 | 148 | 136 | 287 |
| | AFTER STORAGE (60°C, 2 WEEKS) | 179 | 85 | 149 | 293 |
| DECREASE OVER TIME | | NO DECREASE | DECREASE | NO DECREASE | NO DECREASE |

Next, an experiment conducted to evaluate a change in viscosity of the adhesive composition according to cholesterols mixed in the adhesive composition, and results thereof will be described.

### (Experiment 2) Evaluation of Fluidity of Adhesive Composition

### (2-1 Preparation of Sample)

The following three kinds of samples were prepared (Samples A, B, and B'). The Samples A and B are adhesive compositions formed of the same components as those of the above-described samples. Samples A and B in the present experiment are adhesive compositions not laminated on the support layer. Sample B' is an adhesive composition containing 5% by mass of cholesterols relative to the total amount of 100% by mass of the adhesive composition.

All adhesive compositions contain the oxybutynin hydrochloride as the medication and also contain lipophilic adhesive base. Table 3 shows a composition table of each adhesive composition. In the table, "Other additives" are additives including a component regulating absorbability of the medication, a component regulating adhesiveness, or the like.

**[Table 3]**

| COMPONENTS [% BY MASS] | SAMPLE A | SAMPLE B | SAMPLE B' |
|---|---|---|---|
| OXYBUTYNIN HYDROCHLORIDE | 15.0 | 15.0 | 15.0 |
| SIS BLOCK COPOLYMER | 17.2 | 10.2 | 9.9 |
| TACKIFYING RESIN | 40.1 | 38.1 | 37.0 |
| LIQUID PARAFFIN | 14.3 | 13.6 | 13.2 |
| CHOLESTEROL | 0.0 | 3.0 | 5.0 |
| OTHER ADDITIVES | 13.4 | 20.1 | 19.9 |
| TOTAL | 100 | 100 | 100 |

### (2-2 Experimental Procedure)

The viscosity was calculated by measuring a flow rate of each sample at 65°C by using a constant test force extrudable capillary rheometer (flow tester (Shimadzu Corporation)).

### (2-3 Results)

The results are shown in Table 4. As a result of measuring the viscosity at 65°C, it was confirmed that when mixing the cholesterols, the viscosity of the adhesive composition remarkably decreased.

**[Table 4]**

| | SAMPLE A | SAMPLE B | SAMPLE B' |
|---|---|---|---|
| VISCOSITY AT 65°C [Pa·S] | 1210 | 204 | 169 |

Next, an experiment conducted to evaluate an effect of releasability of a patch depending on differences such as cholesterols inclusion, and results thereof will be described.

### (Experiment 3) Releasability Evaluation

### (3-1 Preparation of Sample)

The three kinds of prepared samples (Samples A, B, and C) were prepared using the same components and methods as those of the samples (Samples A, B, and C) used in Experiment 1.

### (3-2 Experimental Procedure)

A release rate of the medication was measured by a rotary cylinder method described in Release Test of United States Pharmacopeia. Measurement was performed after preparation of a sample patch (initial state) and after storage at 60°C for 2 weeks by being hermetically wrapped in an aluminum laminate packaging material (after storage), as in Experiment 1. For the two measurements with different conditions, separate samples were used.

### (3-3 Results)

FIG. 2 shows measurement results of Sample A. It was observed that as the release time elapsed from the initial state, the release rate of the medication increased. It was observed that, the release rate of the medication after storage was slightly higher than that in the initial state.

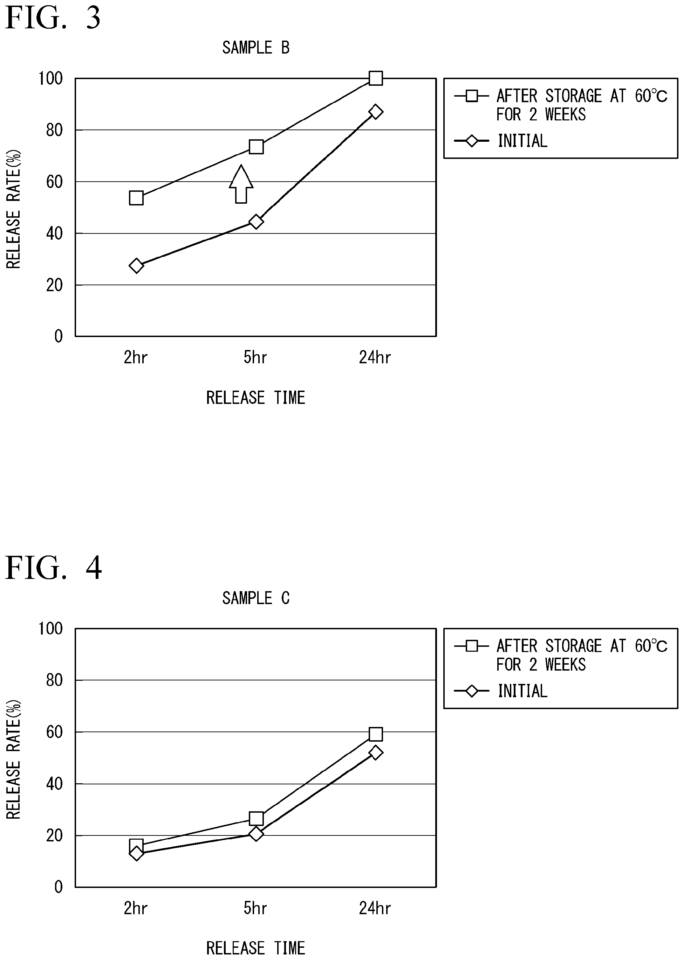

FIG. 3 shows the measurement results of Sample B. In Sample B containing cholesterols, in an initial stage, the release rate of the medication was maintained at almost the same rate as the release rate of the medication of Sample A containing no cholesterols. On the other hand, it was observed that, after storage, the release rate of the medication increased by approximately 20%, compared to the measurement results of Sample A. It is considered that the decrease in viscosity of the adhesive composition according to cholesterols mixing, which was observed in Experiment 2, influences the change of the releasability of the medication over time.

FIG. 4 shows the measurement results of Sample C. It was observed that the release rate of the medication was changed little between the initial state and after storage. It is considered that when using the support layer 11 having the water-repellent surface 110, it is possible to suppress an increase in the release rate of the medication according to cholesterols mixing.

Next, the skin permeability in a case where the support layer 11 formed of the base fabric includes the water-repellent surface 110 was evaluated. For the skin permeability evaluation, an in vitro test using the skin was used.

### (Experiment 4) Skin Permeability Evaluation

### (4-1 Preparation of Sample)

The two kinds of prepared samples (Samples A and C) were prepared using the same components and methods as those of the samples (Samples A and C) used in Experiment 1.

### (4-2 Experimental Procedure)

Dorsal skin of a hairless mouse was sampled, a patch was applied to a stratum corneum side of the skin, and set on a flow-through type Franz cell (permeation area of 3 cm²) such that a dermis side was on a receptor tank side, and a temperature of the cell was kept to 32°C.

Next, while replacing the receptor tank of the cell with a physiological saline solution at a constant flow rate, a bath solution was collected at predetermined time intervals, and a medication concentration in each solution was quantitated by high-performance liquid chromatography. From the quantification result, a maximum permeation rate Jmax of the medication was calculated.

### (4-3 Results)

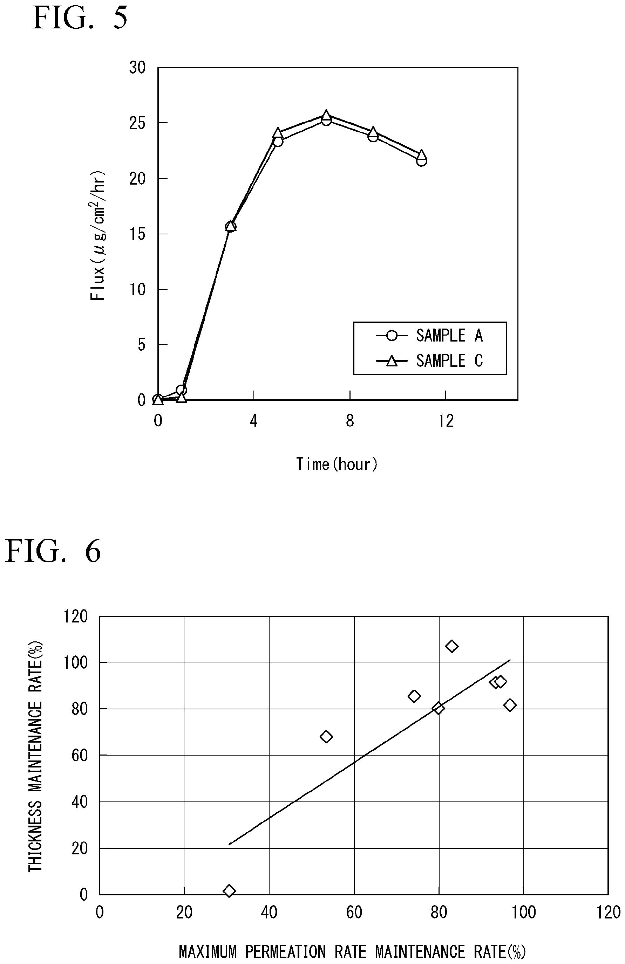

The results are shown in Table 5. A vertical axis of the graph of FIG. 5 represents an average value of the permeation rate of the medication, which was calculated from the results of three experiments. The maximum permeation rates Jmax among the calculated permeation rates of the medication are shown in Table 5. In Samples A and C, as shown in FIG. 5 and Table 5, there were almost no differences in the maximum values and changes over time. That is, it was shown that even when the support layer 11 had the water-repellent surface 110 and the adhesive layer 12 was laminated on the water-repellent surface 110, the skin permeability of the medication was little affected.

**[Table 5]**

| | SAMPLE A | SAMPLE C |
|---|---|---|
| MAXIMUM PERMEATION RATE Jmax [µg/cm²/hr] | 25.2 | 25.8 |

As above, in the patch 1 having cholesterols, in which an effect of reducing the skin irritation is recognized, mixed therein, it was shown that in a case where the support layer 11 formed of the base fabric includes the water-repellent surface 110 at least on one side in a thickness direction thereof, even in a case of being stored under severe conditions, it is possible to reduce changes in the adhesion and the releasability of the medication. In addition, it was also shown that the skin permeability of the medication was little affected.

Next, an experiment conducted to evaluate skin permeability depending on differences such as a kind or a concentration of the water-repellent agent used for the water-repellent surface 110 in the support layer 11, and results thereof will be described.

### (Experiment 5) Evaluation of Skin Permeability Depending on Difference of Water-repellent surface

### (5-1 Preparation of Sample)

Eight kinds of samples were prepared as shown in Tables 6 and 7 (Samples E, F, G, H, I, J, K, and L). Sample E is a patch in which an adhesive composition containing 3% by mass of cholesterols relative to the total amount of 100% by mass of the adhesive composition was laminated on the support layer that has no water-repellent surface 110 and was formed of the base fabric. On the other hand, each of Samples F, G, H, I, J, K, and L is a patch 1 in which the adhesive composition containing 3% by mass of cholesterols relative to the total amount of 100% by mass of the adhesive composition was laminated on the water-repellent surface 110, the water-repellent surface 110 being provided on the support layer 11 formed of the base fabric.

For the water-repellent surface 110 of each of Samples F, G, H, I, and J, AG84 was used as the water-repellent agent. The AG84 contains a fluorine-containing compound.

For the water-repellent surface 110 of each of Samples K and L, NK Guard S was used as the water-repellent agent. The NK Guard S contains a fluorine-containing compound.

All adhesive compositions contain the oxybutynin hydrochloride as the medication and also contain lipophilic adhesive base. Table 7 shows a composition table of each adhesive composition. In the table, "Other additives" are additives including a component regulating absorbability of the medication, a component regulating adhesiveness, or the like.

**[Table 6]**

| | SAMPLES | | | | | | | |
|---|---|---|---|---|---|---|---|---|
| | E | F | G | H | I | J | K | L |
| KINDS OF WATER-REPELLENT AGENT | NONE | AG84 | | | | | NK GUARD S | |
| WATER-REPELLENT AGENT CONCENTRATION (%) | 0 | 0.5 | 2 | 2 | 4 | 6 | 4 | 6 |

**[Table 7]**

| COMPONENTS [% BY MASS] | SAMPLE E | SAMPLES F, G, H, I, J, K, AND L |
|---|---|---|
| OXYBUTYNIN HYDROCHLORIDE | 15.0 | 15.0 |
| SIS BLOCK COPOLYMER | 10.2 | 10.2 |
| TACKIFYING RESIN | 38.2 | 38.2 |
| LIQUID PARAFFIN | 11.6 | 13.6 |
| CHOLESTEROL | 3.0 | 3.0 |
| OTHER ADDITIVES | 22.0 | 20.0 |
| TOTAL | 100 | 100 |

### (5-2 Experimental Procedure)

With respect to the eight kinds of samples, maximum permeation rates Jmax of the medication were calculated by the method as in Experiment 4. Measurement was performed after preparation of a sample patch (initial state) and after storage at 60°C for 1 week by being hermetically wrapped in an aluminum laminate packaging material (after storage). For the two measurements with different conditions, separate samples were used.

### (5-3 Results)

The results are shown in Table 8. After storage, the maximum permeation rate of Sample E having no water-repellent surface remarkably decreased. In Samples F, G, H, I, J, K, and L provided with the water-repellent surface 110, the maximum permeation rate after storage generally improved more than Sample E. All ratios of the maximum permeation rates after storage to those of the initial state (hereinafter referred to as "maximum permeation rate maintenance rate") exceed 50%.

However, among Samples F, G, H, I, J, K, and L provided with the water-repellent surface 110, the maximum permeation rate maintenance rate of Sample F in which the water-repellent agent concentration of the water-repellent surface 110 was 0.5% is the lowest. It is considered that when increasing the water-repellent agent concentration of the water-repellent surface 110, it is possible to improve the maximum permeation rate maintenance rate.

**[Table 8]**

| | | SAMPLES | | | | | | | |
|---|---|---|---|---|---|---|---|---|---|
| | | E | F | G | H | I | J | K | L |
| MAXIMUM PERMEATION RATE Jmax [*µ*g/cm²/hr] | INITIAL STATE | 33. 04 | 21. 68 | 26. 84 | 32. 09 | 27. 71 | 29. 27 | 24. 4 | 23. 71 |
| | AFTER STORAGE (60°C, 1 WEEK) | 10. 12 | 11. 57 | 21. 45 | 29. 97 | 23. 03 | 21. 72 | 23. 61 | 22. 42 |
| MAXIMUM PERMEATION RATE MAINTENANCE RATE(%) | | 30. 6 | 53. 4 | 79. 9 | 93. 4 | 83. 1 | 74. 2 | 96. 8 | 94. 6 |

Next, an experiment conducted to evaluate the skin permeability in a case of long-term storage, and results thereof will be described.

### (Experiment 6) Evaluation of Skin Permeability Depending on Difference of Storage Term

### (6-1 Preparation of Sample)

Two kinds of samples were prepared as shown in Tables 9 and 10 (Samples M and N). Sample M is a patch in which an adhesive composition containing 3% by mass of cholesterols relative to the total amount of 100% by mass of the adhesive composition was laminated on the support layer that has no water-repellent surface 110 and was formed of the base fabric. On the other hand, Sample N is a patch 1 in which the adhesive composition containing 3% by mass of cholesterols relative to the total amount of 100% by mass of the adhesive composition was laminated on the water-repellent surface 110, the water-repellent surface 110 being provided on the support layer 11 formed of the base fabric.

For the water-repellent surface 110 of Sample N, Asahi Guard E060 was used as the water-repellent agent. The Asahi Guard E060 contains a fluorine-containing compound.

All adhesive compositions contain the oxybutynin hydrochloride as the medication and also contain lipophilic adhesive base. Table 10 shows a composition table of each adhesive composition. In the table, "Other additives" are additives including a component regulating absorbability of the medication, a component regulating adhesiveness, or the like.

**[Table 9]**

| | SAMPLE M | SAMPLE N |
|---|---|---|
| KINDS OF WATER-REPELLENT AGENT | NONE | ASAHI GUARD E060 |
| WATER-REPELLENT AGENT CONCENTRATION (%) | 0 | 4 |

**[Table 10]**

| COMPONENTS [% BY MASS] | SAMPLE M | SAMPLE N |
|---|---|---|
| OXYBUTYNIN HYDROCHLORIDE | 10.0 | 10.0 |
| SIS BLOCK COPOLYMER | 11.4 | 11.4 |
| TACKIFYING RESIN | 43.0 | 43.0 |
| LIQUID PARAFFIN | 15.3 | 15.3 |
| CHOLESTEROL | 3.0 | 3.0 |
| OTHER ADDITIVES | 17.3 | 17.3 |
| TOTAL | 100 | 100 |

### (6-2 Experimental Procedure)

With respect to the two kinds of samples, maximum permeation rates Jmax of the medication were calculated by the method as in Experiment 4. Measurement was performed after preparation of a sample patch (initial state) and after storage at 60°C for 1 month by being hermetically wrapped in an aluminum laminate packaging material (after storage). For the two measurements with different conditions, separate samples were used.

### (6-3 Results)

The results are shown in Table 11. After storage, the maximum permeation rate of Sample M having no water-repellent surface remarkably decreased. In Sample N provided with the water-repellent surface 110, the maximum permeation rate after storage improved more than Sample E. It is considered that even in a case of long-term storage of one month, the maximum permeation rate of the medication can be maintained to a certain extent by providing the water-repellent surface 110.

**[Table 11]**

| | | SAMPLE M | SAMPLE N |
|---|---|---|---|
| MAXIMUM PERMEATION RATE Jmax [µg/cm²/hr] | INITIAL STATE | 19. 77 | 18.3 |
| | AFTER STORAGE (60°C, 1 MONTH) | 6. 39 | 19. 4 |
| MAXIMUM PERMEATION RATE MAINTENANCE RATE(%) | | 32.3 | 106.0 |

Next, an experiment conducted to evaluate the adhesive layer after storage, and results thereof will be described.

### (Experiment 7) Evaluation of Adhesive layer After Storage

### (7-1 Preparation of Sample)

The samples were prepared using the same components and methods as those of the eight kinds of samples (Samples E, F, G, H, I, J, K, and L) in Experiment 5.

### (7-2 Experimental Procedure)

A thickness of the adhesive layer was measured after preparation of a sample patch (initial state) and after storage at 60°C for 1 week by being hermetically wrapped in an aluminum laminate packaging material (after storage).

### (7-3 Results)

The results are shown in Table 12. After storage, the thickness of the adhesive layer of Sample E having no water-repellent surface remarkably decreased. In Samples F, G, H, I, J, K, and L provided with the water-repellent surface 110, the thickness of the adhesive layer after storage is thicker than that of Sample E. All ratios of the thicknesses of the adhesive layer after storage to those of the initial state (hereinafter, referred to as "thickness maintenance rate") exceed 50%.

It is considered that, when the cholesterols are mixed in the adhesive layer, the fluidity of the adhesive layer increases, the adhesive layer encroaches on the support layer, and the thickness of the adhesive layer decreases. On the other hand, it is considered that, when providing the water-repellent surface 110 on the support layer 11 as in Samples F, G, H, I, J, K, and L, it is possible to maintain the thickness of the adhesive layer 12 to a certain extent.

**[Table 12]**

| | | SAMPLES | | | | | | | |
|---|---|---|---|---|---|---|---|---|---|
| | | E | F | G | H | I | J | K | L |
| THICKNESS [µm] | INITIAL STATE | 98.5 | 102. 1 | 118.8 | 118.0 | 110.9 | 104. 1 | 102. 1 | 111. 2 |
| | AFTER STORAGE (60°C, 1 WEEK) | 1.4 | 69.3 | 95.0 | 107.7 | 118.4 | 88. 7 | 83. 1 | 101.8 |
| THICKNESS MAINTENANCE RATE(%) | | 1.4 | 67.8 | 80.0 | 91. 2 | 106.8 | 85. 2 | 81.4 | 91. 5 |

FIG. 6 shows a correlation between the "thickness maintenance rate" and the "maximum permeation rate maintenance rate" measured in Experiment 5 of the eight kinds of samples (Samples E, F, G, H, I, J, K, and L). As shown in FIG. 6, the higher the thickness maintenance rate, the higher the maximum permeation rate maintenance rate. It is considered that, when providing the water-repellent surface 110 and reducing the encroachment of the adhesive layer 12 on the support layer 11, it is possible to maintain the maximum permeation rate of the medication and it is more possible to maintain the skin permeability. That is, it is considered that it is effective to reduce encroachment of the adhesive layer 12 on the support layer 11 by providing the water-repellent surface 110, in order to reduce the change in the releasability of the medication over time.

### Industrial Applicability

The present invention is applied to a patch (transdermal absorption preparation) that contains oxybutynin.

### Reference Signs List

- 1: Patch (Oxybutynin-containing transdermal absorption preparation)
- 11: Support layer
- 12: Adhesive layer
- 20: Releasable coating
- 110: Water-repellent surface

## Claims

1. An oxybutynin-containing transdermal absorption preparation, which is a patch (1) comprising:
a support layer (11) that is formed of a base fabric; and
an adhesive layer (12) laminated on one side of the support layer (11),
wherein the adhesive layer (12) includes an adhesive composition containing oxybutynin or a pharmaceutically acceptable salt thereof, cholesterols, and a rubber adhesive base,
**characterized in that**
the support layer (11) has a water-repellent surface (110) formed by a water-repellent treatment with a fluorine-containing compound in at least one side thereof in a thickness direction.

2. The oxybutynin-containing transdermal absorption preparation according to Claim 1,
wherein the oxybutynin is oxybutynin hydrochloride.

3. The oxybutynin-containing transdermal absorption preparation according to any one of Claims 1 to 2,
wherein the base fabric is a knitted fabric.

4. The oxybutynin-containing transdermal absorption preparation according to any one of Claims 1 to 3,
wherein the rubber adhesive base contains a styrene-isoprene-styrene block copolymer.

5. The oxybutynin-containing transdermal absorption preparation according to any one of Claims 2 to 4,
wherein a content percentage of the oxybutynin is 4% to 50% by mass relative to the adhesive composition, and
cholesterol is used for the cholesterols, and a content percentage thereof is 0.05 to 10% by mass relative to the adhesive composition.

## Patentansprüche

1. Eine Oxybutynin-haltige transdermale Absorptionszubereitung, bei welcher es sich um ein Pflaster (1) handelt, umfassend:
eine Trägerschicht (11), die aus einem Grundgewebe gebildet ist; und
eine Klebstoffschicht (12), die auf eine Seite der Trägerschicht (11) laminiert ist,
wobei die Klebstoffschicht (12) eine Klebstoffzusammensetzung enthält, die Oxybutynin oder ein pharmazeutisch akzeptables Salz davon, Cholesterin-Verbindungen und eine Gummiklebemasse enthält,
**dadurch gekennzeichnet, dass**
die Trägerschicht (11) eine wasserabweisende Oberfläche (110) aufweist, die durch eine wasserabweisende Behandlung mit einer fluorhaltigen Verbindung auf in einer Dickenrichtung gesehen mindestens einer ihrer Seiten gebildet ist.

2. Die Oxybutynin-haltige transdermale Absorptionszubereitung gemäß Anspruch 1,
wobei es sich bei dem Oxybutynin um Oxybutyninhydrochlorid handelt.

3. Die Oxybutynin-haltige transdermale Absorptionszubereitung gemäß einem der Ansprüche 1 bis 2,
wobei das Grundgewebe ein Maschengewebe ist.

4. Die Oxybutynin-haltige transdermale Absorptionszubereitung gemäß einem der Ansprüche 1 bis 3,
wobei die Gummiklebemasse ein Styrol-Isopren-Blockcopolymer enthält.

5. Die Oxybutynin-haltige transdermale Absorptionszubereitung gemäß einem der Ansprüche 2 bis 4,
wobei ein Gehalt des Oxybutynins 4 bis 50 Masseprozent bezüglich der Klebstoffzusammensetzung beträgt und
Cholesterin as Cholesterin-Verbindung verwendet wird, wobei ein Gehalt des Cholesterins 0,05 bis 10 Masseprozent bezüglich der Klebstoffzusammensetzung beträgt.

## Revendications

1. Une préparation pour absorption transdermique contenant de l'oxybutynine, laquelle est un patch (1) comprenant :
une couche de support (11) formée d'un tissu de base ; et
une couche adhésive (12) laminée sur une face de la couche de support (11),
dans laquelle la couche adhésive (12) comprend une composition adhésive comprenant de l'oxybutynine ou un sel pharmaceutiquement acceptable de l'oxybutynine, des composés de cholestérol et une base adhésive en caoutchouc,
**caractérisée en ce que**
la couche de support (11) présente une surface hydrofuge (110) formée par un traitement hydrofuge par un composé contenant du fluor sur au moins un de ses faces dans une direction d'épaisseur.

2. La préparation pour absorption transdermique contenant de l'oxybutynine selon la revendication 1,
dans laquelle l'oxybutynine est le chlorhydrate d'oxybutynine.

3. La préparation pour absorption transdermique contenant de l'oxybutynine selon l'une des revendications 1 à 2,
dans laquelle le tissu de base est un tissu tricoté.

4. La préparation pour absorption transdermique contenant de l'oxybutynine selon l'une des revendications 1 à 3,
dans laquelle la base adhésive en caoutchouc contient un copolymère à blocs styrène-isoprène-styrène.

5. La préparation pour absorption transdermique contenant de l'oxybutynine selon l'une des revendications 2 à 4,
dans laquelle le pourcentage de l'oxybutynine est compris entre 4 % et 50 % en masse par rapport à la composition adhésive, et
cholestérol est utilisé comme le composé de cholestérol, et son pourcentage de contenu est de 0,05 à 10 % en masse par rapport à la composition adhésive.
